# EUROPÄISCHE PATENTSCHRIFT

(11) **EP 3 458 196 B1**
(45) Veröffentlichungstag und Bekanntmachung des Hinweises auf die Patenterteilung: **22.05.2024**
(21) Anmeldenummer: 17721387.3
(22) Anmeldetag: 04.05.2017
(51) Int. Cl.: B04B 1/08, B04B 15/06

(54) **VERFAHREN ZUR THERMISCHEN DESINFIZIERUNG EINER ZENTRIFUGE**
METHOD FOR THERMALLY DISINFECTING A CENTRIFUGE
PROCÉDÉ DE DÉSINFECTION THERMIQUE D'UNE CENTRIFUGE

(30) Priorität: 18.05.2016 DE 102016109086
(43) Veröffentlichungstag der Anmeldung: 27.03.2019
(73) Patentinhaber: GEA Mechanical Equipment GmbH, 59302 Oelde (DE)
(72) Erfinder: BATHELT, Thomas, 59302 Oelde (DE); ESSELING, Dirk, 59302 Oelde (DE)
(74) Vertreter: Specht, Peter
(86) Internationale Anmeldenummer: PCT/EP2017/060715
(87) Internationale Veröffentlichungsnummer: WO 2017/198475

(56) Entgegenhaltungen:
- WO-A2-2004/058173
- DE-A1- 1 910 211
- DE-A1- 2 656 271
- DE-A1- 4 108 538
- US-A- 3 476 310

## Beschreibung

Die Erfindung bezieht sich auf ein Verfahren zur thermischen Desinfizierung - d.h. zur Keimreduzierung der Oberflächen der Durchflusswege - einer eine drehbare Trommel aufweisenden Zentrifuge, die zur zentrifugalen Verarbeitung eines Produktes dient.

Bei der zentrifugalen Verarbeitung von bestimmten Produkten, d.h. bei bestimmten Anwendungen, ist es erforderlich, die Zentrifuge nicht nur zu reinigen - beispielsweise mit Hilfe eines CIP-Verfahrens - sondern auch zur Keimabtötung zu desinfizieren. Hierzu wird in der Regel eine Dampfsterilisation eingesetzt.

Die DE 26 56 271 A1 offenbart beispielsweise ein Verfahren zur thermischen Desinfizierung einer Zentrifuge.

Die DE 1 910 211 A1 offenbart einen mit Heißdampf sterilisierbaren Separator bei stillstehender Trommel.

Weiterer Stand der Technik findet sich in der DE 41 08 538 A1, der US 3, 476, 310 A und der WO 2004/058 173 A2.

Bei der Dampfsterilisation wird heißer Wasserdampf unter Druck durch die Zentrifuge geleitet. Dazu wird bei Zentrifugen Wasserdampf mit einer Temperatur von 121 °C bzw. 134 °C eingesetzt. Die Zentrifuge wird daher unter Druck mit Dampf beschickt. Üblicherweise findet die Dampfsterilisation bei 127°C und 2,5 bar statt.

Aus der Dampfsterilisation mit ihren hohen Drücken resultiert die Notwendigkeit, einen "Druckbehälter" (u.a. als Trommel und als Zu- und Ableitungen) einzusetzen, welcher den lokalen anzuwendenden Regelwerken entspricht. Diese Regelwerke sind üblicherweise der "ASME boiler and pressure vessel code" oder die "Druckgeräterichtlinie" (mit Ausführung nach AD-Regelwerk). Hierfür sind entsprechende Berechnungen, Werkstoffe, Nachweise usw. erforderlich, welche die Kosten des Separators signifikant erhöhen.

Die Erfindung hat ausgehend von diesem Stand der Technik die Aufgabe, ein vereinfachtes Verfahren zur Keimreduzierung von Separatoren zu schaffen.

Die Erfindung löst diese Aufgabe durch den Gegenstand des Anspruchs 1. Geschaffen wird ein Verfahren zur thermischen Desinfizierung - d.h. zur Keimreduzierung - von Zentrifugen, die zur zentrifugalen Verarbeitung eines Produktes dienen, wobei das Desinfizieren - nicht während der eigentlichen Verarbeitung des Produktes sondern quasi in einer Verarbeitungspause - mit Dampf erfolgt, der nicht unter erhöhtem Druck steht bzw. der unter Atmosphärendruck steht.

Ein besonderer Vorteil dieses Verfahrens ist darin zu sehen, dass kein Druckbehälter erforderlich ist, d.h. insbesondere die Trommel und deren Zu- und Ableitungen und/oder die Haube und der Feststofffänger müssen lediglich für einen Betrieb unter Atmosphärendruck ausgelegt sein. Sehr einfach wird das Verfahren, wenn als Dampf Wasserdampf unter Atmosphärendruck verwendet wird. Es erfolgt eine thermische Desinfektion, bei der eine Erhitzung der zu desinfizierenden Objekte zur Keimreduzierung unter Dampfzufuhr unter Atmosphärendruck durchgeführt wird. Hierdurch wird die Keimzahl in einem definierten Maß verringert.

Geeignet ist das erfindungsgemäße Verfahren mit seinen Varianten insbesondere für Anwendungen im Bereich der Biotechnologie / Pharmazie, bei denen eine thermische Sanitisierung/Desinfizierung anstelle einer thermischen Sterilisierung ausreicht. Dies bedeutet, dass lediglich eine Abtötung von Keimen bei Temperaturen erforderlich ist, die bei Atmosphärendruck mit dem verwendeten Dampf erreichbar sind. Bei solchen Anwendungen erspart die Erfindung die ansonsten erforderliche Druckbehälterauslegung.

Es ist weiter vorteilhaft, wenn der Dampf unter Atmosphärendruck durch den (gesamten) Produktweg der Zentrifuge geleitet wird. Der Weg, den das Produkt und seine einzelnen Phasen in, durch den und aus dem Separator nehmen, wird dabei und nachfolgend als der Produktweg bezeichnet. Dazu gehören somit der Zulauf mit dem Zulaufrohr, der Verteiler, der Schleuderraum mit dem Trenntellerstapel, der Ablauf mit der Schälscheibe und der Ablaufleitung sowie ggf. (bei einer Feststoffabtrennung) die Austrittsöffnungen, der Haubeninnenraum mit einem Feststofffänger.

Der Begriff "Sanitisierung" bezeichnet im Rahmen dieser Anmeldung eine drucklose thermische Desinfizierung, also eine "Keimreduzierung". Im Gegensatz dazu wird bei einer Dampf-Sterilisierung eine Sterilität erreicht. Das Ziel der erfindungsgemäßen "Sanitisierung" ist mithin eine (anwendungsspezifisch hinreichende) Reduzierung der relevanten Keime und Mikroorganismen.

Die Trommel rotiert während der Durchleitung des Dampfes mit einer vorgegebenen Drehzahl. Die Dampfdurchleitung während der Rotation der Trommel sorgt für eine optimierte Verteilung des Dampfes und für eine verbesserte Temperaturführung und für eine Abdichtung zum Getrieberaum (Antriebsraum). Die Drehzahl der Trommel wird auf den niedrigsten möglichen Wert abgesenkt, bei dem ein Eindringen von Dampf in den Antriebsbereich noch verhindert wird.

Eine Messung der effektiv erreichten Temperaturen (T) an relevanten Messstellen (z.B. Zulauf, Produkt-Ablauf, Kondensat-Ablauf, Haube, Fänger) ist auf einfache Weise mit Temperatursensoren möglich. Hierdurch wird überwacht, wie lange wo im Durchflussweg bzw. an den Oberflächen des Durchflussweges welche Temperatur bei der Sanitisierung erreicht worden ist. Ergänzend ist es vorteilhaft, mit einem Feuchtesensor den Feuchtegehalt im Antriebsraum zu detektieren und/oder mit einem Detektor zur Wasserdetektion in Schmieröl den Wassergehalt in einem Schmiermittelsystem. Werden bei der thermischen Sanitisierung vorgegebene Grenzen erreicht oder überschritten, wird eine Warnmeldung ausgegeben und/oder die Sanitisierung gestoppt.

Weitere vorteilhafte Ausgestaltungen sind den übrigen Unteransprüchen zu entnehmen.

Nachfolgend wird die Erfindung anhand eines Ausführungsbeispiels unter Bezug auf die Figuren näher beschrieben. Es zeigen:

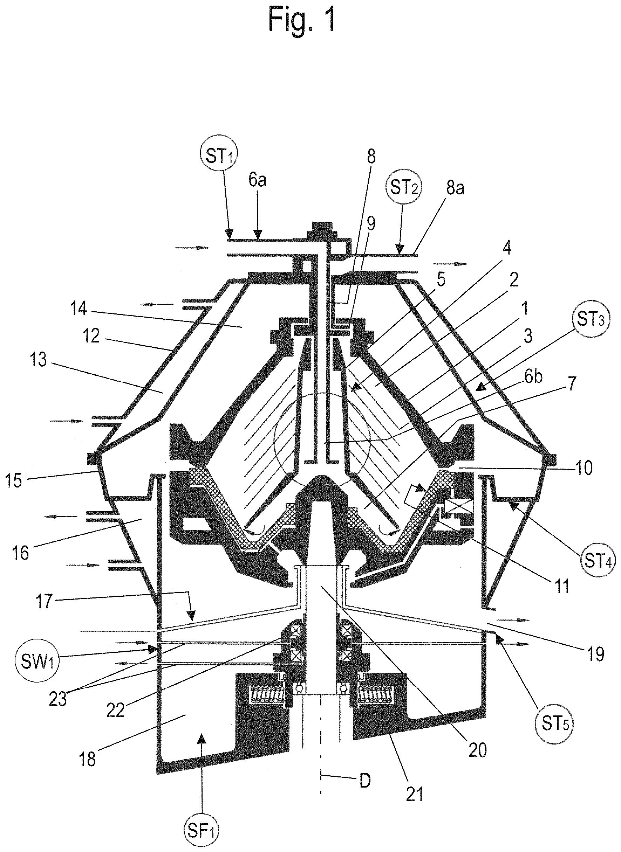

- Fig. 1: eine schematische Darstellung eine Separators ohne Darstellung eines Antriebsmotors.

Fig. 1 zeigt eine Zentrifuge, hier einen Separator, der eine drehbare Trommel 1 aufweist. Diese Trommel 1 ist hier doppel-konisch ausgebildet.

Die Trommel 1 weist eine vertikale Drehachse D auf. In der konischen bzw. hier sogar doppelt-konisch geformten Trommel 1 ist im (hier beispielhaft ebenfalls doppelkonischen ) Trommelinnenraum - auch Schleuderraum 2 genannt - ein Trenntellerstapel 3 aus konischen Trenntellern 4 angeordnet. Die Trennteller 4 sind auf einem Verteilerschaft 5 eines Verteilers angeordnet. Ein Zulauf 6a mit einem in die Trommel ragenden Zulaufrohr 6b dient zur Zuleitung eines zu verarbeitenden Produktes in Verteilerkanäle 7 und aus diesen in den Schleuderraum 2.

In dem Schleuderraum 2 erfolgt eine Klärung des zu verarbeitenden Produktes von Feststoffen sowie ggf. optional eine Trennung in zwei oder mehr Flüssigkeitsphasen unterschiedlicher Dichte.

Zur Ableitung der wenigstens einen Flüssigkeitsphase dienen einer oder mehrere Abläufe 8 für Flüssigkeitsphasen, die beispielsweise mit Schälscheiben 9 versehen sein können und in eine Ablaufleitung 8a münden.

Die Feststoffe werden dagegen durch umfangsverteilte, sich radial erstreckende Austrittsöffnungen 10 vorzugsweise im Bereich des größten Radius/Umfangs der Zentrifugentrommel aus der Zentrifugentrommel 1 nach außen ausgestoßen. Nach einer Variante (hier nicht dargestellt) sind diese Austrittsöffnungen 10 mit Düsen versehen, um die Feststoffe kontinuierlich auszuschleudern. Alternativ ist den Austrittsöffnungen 10 ein diskontinuierlich arbeitender Verschlussmechanismus zugeordnet. Dieser kann einen fluidbetätigten Kolbenschieber 11 aufweisen, der die Austrittsöffnungen 10 in einer Stellung verschließt und in einer anderen Stellung freigibt, so dass eine Feststoffentleerung erfolgen kann.

Die Trommel 1 ist von einer Haube 12 umgeben. Diese Haube 12 ist hier abschnittsweise doppelwandig ausgeführt, so dass sie in diesem Doppelwandabschnitt 13 von einem Fluid durchströmt werden kann.

Zwischen der Trommel 1 und der Haube 12 ist ein Haubeninnenraum 14 ausgebildet, der nach unten hin einen Feststofffänger 15 aufweist oder ausbildet, der zum Auffangen von Feststoff dient, der aus der Trommel 1 ausgestoßen wird. Unterhalb des Feststofffängers 15 ist ein weiterer Doppelwandabschnitt 16 an der Haube 12 ausgebildet, der von einem Fluid durchströmbar ist. Dieses Fluid kann ein Kühlmittel sein (bei der Produktverarbeitung). Es kann aber auch der Dampf sein, um die Haube in diesen Bereichen für eine thermische Desinfektion zu erhitzen.

Unterhalb der Trommel 1 ist eine Membran 17 angeordnet, die den Haubeninnenraum von einem Antriebsraum 18 trennt. An der Membran 17 ist ein Gestellablauf 19 ausgebildet.

Die Trommel 1 ist mittels einer Antriebsspindel 20 drehbar. Diese Antriebspindel 20 ist in einem Maschinengestell 21 in dem Antriebsraum 18 unterhalb der Trommel 1 mit einem oder mehreren Spindellagern 22 drehbar gelagert. Die Trommel 1 ist hier auf ein oberes Ende der Antriebsspindel 20 aufgesetzt. Ein Schmierstoffsystem 23 dient zur Versorgung der Spindellager 22 mit Schmierstoff.

Mit wenigstens einem oder vorzugsweise einer Mehrzahl von Sensoren können an verschiedenen Stellen Betriebsparameter gemessen werden. Diese Sensoren sind an eine (hier nicht dargestellte) Steuerungseinheit zur Steuerung des Betriebs des Separators und auch des Sanitisierungsverfahrens angeschlossen. Hierzu weist sie ein Computerprogramm auf, welches den Ablauf des Sanitisierungsverfahrens steuert.

Die Sensoren umfassen hier
- einen oder mehrere Temperatursensoren ST1 bis STn,
- einen oder mehrere Feuchtesensoren SF1 und/oder einen
- einen oder mehrere Sensoren SW1 zur Detektion von Wasser im Schmieröl des Schmiersystems 23.

Der Weg, den das Produkt und seine einzelnen Phasen in, durch den und aus dem Separator nehmen, wird nachfolgend als der Produktweg bezeichnet. Dazu gehören somit der Zulauf 6a mit dem Zulaufrohr 6b, der Verteiler 7, der Schleuderraum 2 mit dem Trenntellerstapel 3, der Ablauf 8 mit der Schälscheibe 9, die Ablaufleitung 8a, sowie die Austrittsöffnungen 10 und der Haubeninnenraum 14 mit dem Feststofffänger 15.

Mit wenigstens dem einem oder vorzugsweise mehreren Temperatursensoren ST1 bis STn (hier beispielhaft fünf) kann die Temperatur an verschiedenen Stellen und/oder in Bereichen an Oberflächen des Produktweges des Separators gemessen werden. Vorzugsweise sind dazu einer oder mehrere Temperatursensoren ST1 bis STn zur Messung der entsprechenden Temperatur an einem oder mehreren folgender Stellen vorgesehen: An dem Zulauf 6a mit dem Zulaufrohr 6b, an dem Gestellablauf 19, an der Ablaufleitung 8a und/oder an/in dem Haubeninnenraum 14 und/oder an oder in dem Feststofffänger 15.

Der Feuchtesensor SF1 ist hier im Antriebsraum 18 angeordnet, um die Luftfeuchtigkeit im Antriebsraum/-bereich 18 zu überwachen.

Der Sensor SW1 zur Messung von Sensierung von Wasser im Schmieröl ist dem Schmierstoffsystem 23 zugeordnet, um einen/den Wasseranteil im Schmieröl zu bestimmen.

Der Separator und dessen Trommel 1 ist/sind vorzugsweise für einen kontinuierlichen Betrieb - d.h. die kontinuierliche und nicht chargenweise Verarbeitung eines Produktes - ausgelegt. Bei dieser Verarbeitung wird ein zu verarbeitendes Produkt in verschieden dichte Phasen getrennt. Beispielsweise kann eine Flüssigkeit geklärt werden und/oder in zwei verschieden dichte Flüssigkeitsphasen getrennt werden. Zudem ist es derart auch möglich, aus einem Ausgangsprodukt eine Feststoffphase als Wertprodukt aufzukonzentrieren und von einer leichteren Phase zu trennen.

Zum Reinigen kann eine CIP-Reinigung durchgeführt werden (cleaning in place), wobei die Zentrifuge mit Flüssigkeiten, wie zum Beispiel Säuren, Laugen oder Wasser gereinigt und durchspült wird.

Um eine weitergehende Inaktivierung von Keimen und Sporen durchzuführen, ist zudem vorgesehen, zur Desinfizierung Dampf durch den Produktweg des Separators zu leiten, der nicht unter erhöhtem Druck sondern unter Atmosphärendruck steht. Primär geeignet ist dieses Desinfizierungsverfahren für Anwendungen im Bereich der Biotechnologie / Pharmazie, bei denen eine thermische Sanitisierung/Desinfizierung anstelle einer thermischen Sterilisierung ausreicht. Das Ziel der "Sanitisierung" ist mithin eine (anwendungsspezifisch hinreichende) Reduzierung der relevanten Keime und Mikroorganismen.

Für eine solche Keimreduzierung/Inaktivierung sind in der nachfolgenden Tabelle 1 einige erforderliche Temperaturen und Zeiten angegeben.

**Tabelle 1**

| | Hitzeresistenz | | | | | |
|---|---|---|---|---|---|---|
| Resistenzstufe | | Organismus/ Krankheitserreger | | Temperatur (°C) | | Zeit (min) |
| | I | | Pathogene Streptokocken, Listerien, Polioviren | | 61,5 | 30 |
| | II | | die meisten vegetativen Bakterien, Hefen, Schimmelpilze, alle Viren außer Hepatitis-B | | 80 | 30 |
| | III | | Hepatitis-B-Viren, die meisten Pilzsporen | | 100 | 5-30 |
| | IV | | Bacillus-anthracis-Sporen | | 105 | 5 |
| | V | | Bacillusstearothermophilus-Sporen | | 121 | 15 |
| | VI | | Prionen | | 132 | 60 |

Erfindungsgemäß erfolgt eine Keimreduzierung - d-h. ein thermisches Sanitisieren - mit Dampf, der nicht unter erhöhtem Druck steht bzw. der unter Atmosphärendruck steht. Dies genügt beispielsweise zum Abtöten der Keime der Hitzeresistenzstufen I bis III, falls die Sanitisierung bei einem atmosphärischen Druck von 1bar erfolgt

Da ein druckloses Dampfaufgeben zum Sanitisieren der Zentrifuge erfolgt, ist kein Druckbehälter erforderlich, d.h. die Trommel 1 und deren Zu- und Ableitungen 6,6a, 8, 8a sowie der Haubenraum 14 und der Feststofffänger 15 müssen lediglich für einen Betrieb unter Atmosphärendruck ausgelegt sein.

Der Weg, den das Produkt und seine einzelnen Phasen in, durch den und aus dem Separator nehmen, wird nachfolgend als der Produktweg bezeichnet. Dazu gehören somit der Zulauf 6 mit dem Zulaufrohr 6a, der Verteiler, der Schleuderraum 2 mit dem Trenntellerstapel 3, der Ablauf 8 mit der Schälscheibe 9 und die Ablaufleitung 8a sowie ggf. (bei einer Feststoffabtrennung) die Austrittsöffnungen 10, der Haubeninnenraum 14 mit dem Feststofffänger 15.

Um eine weitergehende Inaktivierung von Keimen und Sporen durchzuführen, ist vorgesehen, Dampf durch den gesamten Produktweg der Zentrifuge bzw. hier des Separators zu leiten, der dazu nicht unter erhöhtem Druck sondern unter Atmosphärendruck steht.

Vorzugsweise erfolgt ein Zuführen des Dampfes durch den Zulauf, dann durch den Rotor bzw. den Schleuderraum 2 und dann durch den Ablauf und die Ablaufleitung. Haubenraum 14, den Feststofffänger 15 und ggf. optional eine Schutzhaube.

Derart kann eine geführte Sanitisierung des gesamten produktberührten Bereichs der Zentrifuge erfolgen.

Eine Dampfdurchleitung während einer Rotation der Trommel 1 sorgt für eine verbesserte Verteilung des Dampfes und für eine verbesserte Temperaturführung und für eine Abdichtung zum Getrieberaum (Antriebsraum).

Eine Messung der effektiv erreichten Temperaturen (T) an relevanten Messstellen mit den Sensoren ST1 bis STn ist vorteilhaft möglich. Insofern wird das Verfahren so lange durchgeführt, bis an einem, mehreren oder jedem der Temperatursensoren ST1 bis STn eine vorgegebene Mindesttemperatur für eine vorgegebene Zeitspanne entsprechend der abzutötenden Keime erreicht worden ist. Vorzugseise liegt diese Temberatur und diese Zeit gemäß Tabelle 1 bei mehr als 61,5°C für mehr als 30 min insbesondere bei mehr als 80°C für mehr als 30 min.

Es ist vorteilhaft, mit dem Feuchtesensor SF1 den Feuchtegehalt im Antriebsraum 18 zu detektieren und mit dem Detektor SW1 den Wasseranteil im Schmieröl in einem Schmierstoffsystem 23 zu detektieren.

Werden bei der thermischen Sanitisierung vorgegebene Grenzen für den Feuchtegehalt im Antriebsraum oder den Wasseranteil im Schmieröl erreicht oder überschritten, wird eine Warnmeldung ausgegeben und/oder die Sanitisierung gestoppt.

### Bezugszeichen

| | |
|---|---|
| Trommel | 1 |
| Schleuderraum | 2 |
| Trenntellerstapel | 3 |
| Trennteller | 4 |
| Verteilerschaft | 5 |
| Zulauf | 6a |
| Zulaufrohr | 6b |
| Verteilerkanäle | 7 |
| Ablauf | 8 |
| Ablaufleitung | 8a |
| Schälscheiben | 9 |
| Austrittsöffnungen | 10 |
| Kolbenschieber | 11 |
| Haube | 12 |
| Doppelwandabschnitt | 13 |
| Haubeninnenraum | 14 |
| Feststofffänger | 15 |
| Doppelwandabschnitt | 16 |
| Membran | 17 |
| Antriebsraum | 18 |
| Gestellablauf | 19 |
| Antriebsspindel | 20 |
| Maschinengestell | 21 |
| Spindellager | 22 |
| Schmierstoffsystem | 23 |
| Temperatursensoren | ST1 bis ST5 |
| Feuchtesensoren | SF1 |
| Sensoren | SW1 |
| Drehachse | D |

## Patentansprüche

1. Verfahren zur thermischen Desinfizierung - d.h. zur Keimreduzierung - einer eine drehbare Trommel (1) aufweisenden Zentrifuge, die zur zentrifugalen Verarbeitung eines Produktes dient, wobei die Keimreduzierung mit Dampf erfolgt, der nicht unter erhöhtem Druck steht bzw. der unter Atmosphärendruck steht.
wobei das Durchleiten des Dampfes durch einen Zulauf (6, 6a), Verteilerkanäle 7, durch einen Schleuderraum (2), durch den Trenntellerstapel (3) und durch einen Ablauf (8, 8a) erfolgt, **dadurch gekennzeichnet, dass** die Trommel (1) während der Durchleitung des Dampfes mit einer Drehzahl unterhalb der Betriebszahl während der zentrifugalen Verarbeitung des jeweiligen Produktes gedreht wird, die auf den niedrigsten möglichen Wert eingestellt ist, bei dem ein Eindringen von Dampf in einen Antriebsraum (18) gerade noch verhindert wird, und
dass eine Messung der Luftfeuchtigkeit in dem Antriebsraum (18) erfolgt.

2. Verfahren nach Anspruch 1, **dadurch gekennzeichnet, dass** als Dampf Wasserdampf unter Atmosphärendruck verwendet wird.

3. Verfahren nach Anspruch 1 oder 2, **dadurch gekennzeichnet, dass** der Dampf unter Atmosphärendruck durch einen Produktweg der Zentrifuge geleitet wird.

4. Verfahren nach Anspruch 1, **dadurch gekennzeichnet, dass** das Durchleiten des Dampfes ferner durch Austrittsöffnungen 10, eine Haube (12) und/oder einen Feststofffänger (15) erfolgt.

5. Verfahren nach Anspruch 1 oder 4, **dadurch gekennzeichnet, dass** die Trommel (1) während der Durchleitung des Dampfes mit einer Drehzahl gedreht wird, die unterhalb der Betriebszahl während der zentrifugalen Verarbeitung des jeweils zu verarbeitenden Produktes liegt.

6. Verfahren nach einem der vorstehenden Ansprüche, **dadurch gekennzeichnet, dass** eine Messung der effektiv erreichten Temperaturen (T) an einer oder mehreren Messstellen erfolgt.

7. Verfahren nach einem der vorstehenden Ansprüche, **dadurch gekennzeichnet, dass** eine Messung der effektiv erreichten Temperaturen (T) an einer oder mehreren Messstellen mit einem oder mehreren Temperatursensoren (ST1 - STn) erfolgt, wobei zu den Messstellen gehören:
- der Zulauf (6a),
- die Ablaufleitung (8a),
- der Gestellablauf (19),
- eine Haube (12), und/oder
- ein Feststofffänger (15).

8. Verfahren nach einem der vorstehenden Ansprüche, **dadurch gekennzeichnet, dass** eine Messung des Wasseranteils im Schmieröl in einem Schmierstoffsystem (23) erfolgt.

9. Verfahren nach einem der vorstehenden Ansprüche, **dadurch gekennzeichnet, dass** dieses so lange durchgeführt wird, bis an einem, mehreren oder jedem der Temperatursensoren (ST1 bis STn) eine vorgegebene Mindesttemperatur für eine vorgegebene Zeitspanne erreicht worden ist.

## Claims

1. Method for thermal disinfection - i.e. for germ reduction - of a centrifuge having a rotatable drum (1) which is used for centrifugal processing of a product, wherein the germ reduction is carried out with steam which is not under increased pressure or which is under atmospheric pressure, wherein the steam is passed through an inlet (6, 6a), distribution channels (7), through a centrifugal chamber (2), through the separating disc stack (3) and through an outlet (8, 8a),
**characterized in that**
the drum (1) is rotated during the passage of the steam at a speed below the operating speed during the centrifugal processing of the respective product, which is set to the lowest possible value at which the penetration of steam into a drive chamber (18) is just prevented, and
**in that** the humidity in the drive chamber (18) is measured.

2. Method according to claim 1, **characterized in that** water vapor under atmospheric pressure is used as steam.

3. Method according to claim 1 or 2, **characterized in that** the steam is passed under atmospheric pressure through a product path of the centrifuge.

4. Method according to claim 1, **characterized in that** the steam is also passed through outlet openings 10, a hood (12) and/or a solids trap (15).

5. Method according to claim 1 or 4, **characterized in that** the drum (1) is rotated during the passage of the steam at a speed which is below the operating speed during the centrifugal processing of the respective product to be processed.

6. Method according to one of the preceding claims, **characterized in that** a measurement of the effectively reached temperatures (T) is carried out at one or more measuring points.

7. Method according to one of the preceding claims, **characterized in that** the effectively reached temperatures (T) are measured at one or more measuring points using one or more temperature sensors (ST1-STn), wherein the measuring points include
- the inlet (6a),
- the outlet pipe (8a),
- the frame outlet (19),
- a hood (12), and/or
- a solids trap (15).

8. Method according to one of the preceding claims, **characterized in that** a measurement of the water content in the lubricating oil in a lubricant system (23) is carried out.

9. Method according to one of the preceding claims, **characterized in that** this is carried out until a predetermined minimum temperature has been reached at one, several or each of the temperature sensors (ST1 to STn) for a predetermined period of time.

## Revendications

1. Procédé de désinfection thermique, c'est-à-dire de réduction de la contamination microbienne, d'une centrifugeuse comportant un tambour (1) rotatif qui sert au traitement par centrifugation d'un produit, la réduction de la contamination microbienne étant réalisée à l'aide de vapeur qui n'est pas à une pression augmentée ou qui est à la pression atmosphérique, dans lequel la vapeur est acheminée par une arrivée (6, 6a), des canaux de distribution (7), un bras de centrifugation (2), la pile de disques de séparation (3) et une évacuation (8, 8a), **caractérisé en ce que** le tambour (1) tourne, pendant l'acheminement de la vapeur, à une vitesse de rotation inférieure à la vitesse de rotation en fonctionnement pour le traitement par centrifugation du produit en question, qui est réglée à la valeur la plus basse possible à laquelle la pénétration de vapeur dans un espace d'entraînement (18) est encore tout juste empêchée, et l'humidité de l'air est mesurée dans l'espace d'entraînement (18).

2. Procédé selon la revendication 1, **caractérisé en ce que** la vapeur utilisée est de la vapeur d'eau à la pression atmosphérique.

3. Procédé selon la revendication 1 ou 2, **caractérisé en ce que** la vapeur est amenée à la pression atmosphérique dans la centrifugeuse par un trajet de produit.

4. Procédé selon la revendication 1, **caractérisé en ce que** la vapeur est en outre acheminée à travers des ouvertures de sortie (10), un capot (12) et/ou un collecteur de solides (15).

5. Procédé selon la revendication 1 ou 4, **caractérisé en ce que** le tambour (1) tourne, pendant l'acheminement de la vapeur, à une vitesse de rotation inférieure à la vitesse de rotation en fonctionnement pour le traitement par centrifugation du produit à traiter.

6. Procédé selon l'une des revendications précédentes, **caractérisé en ce que** les températures (T) effectivement atteintes sont mesurées en un ou plusieurs points de mesure.

7. Procédé selon l'une des revendications précédentes, caractérisé en ce les températures (T) effectivement atteintes sont mesurées en un ou plusieurs points de mesure avec une ou plusieurs sondes de température (ST1 - STn), les points de mesure comprenant :
- l'arrivée (6a),
- la conduite d'évacuation (8a),
- l'évacuation du bâti (19),
- un capot (12) et/ou
- un collecteur de solides (15).

8. Procédé selon l'une des revendications précédentes, **caractérisé en ce que** la teneur en eau de l'huile de lubrification est mesurée dans un circuit de lubrifiant (23).

9. Procédé selon l'une des revendications précédentes, **caractérisé en ce qu'**il est exécuté jusqu'à ce qu'une température minimale prédéterminée soit atteinte pendant une durée prédéterminée au niveau d'une, plusieurs ou chacune des sondes de température (ST1 à STn).
